# FASCICULE DE BREVET EUROPEEN

(11) **EP 0 857 465 B1**
(45) Date de publication et mention de la délivrance du brevet: **04.06.2003**
(21) Numéro de dépôt: 98400287.3
(22) Date de dépôt: 09.02.1998
(51) Int. Cl.: A61B 17/86, A61B 17/68

(54) **Dispositif de vis sécable pour plaque d'ostéosynthèse ou pour la coaptation de deux fragments d'os**
Trennbare Vorrichtung für eine Knochenplatte oder zur Fixierung zweier Knochenfragmente
Separable screw for an osteosynthesis plate or for setting two bone fragments

(30) Priorité: 10.02.1997 FR 9701639
(43) Date de publication de la demande: 12.08.1998
(73) Titulaire: S.C.I. DIGO, 2550 Luxembourg (LU)
(72) Inventeur: Diebold, Patrice Francois, 54000 Nancy (FR)
(74) Mandataire: Martin, Didier

(56) Documents cités:
- EP-A- 0 530 160
- EP-A- 0 699 420
- DE-U- 9 017 101
- FR-A- 2 625 430
- US-A- 5 122 132
- US-A- 5 334 204

## Description

La présente invention a pour objet un dispositif de vis sécable pour plaque d'osthéosynthèse ou pour la coaptation de deux fragments d'os.

On connaît d'après le document FR-A-2 625 430 une vis sécable qui comporte une tige filetée prolongée d'une tête solidaire, par l'intermédiaire d'une zone étranglée de moindre résistance à un couple donné, d'un embout serrable dans le mandrin d'un outil électrique de vissage.

Cette vis, si elle permet un vissage direct et rapide au moyen de l'outil de vissage, présente toutefois des inconvénients. En effet, d'une part sa tête fait saillie de la plaque d'osthéosynthèse, et d'autre part, bien qu'elle soit autotaraudeuse et autoforeuse, elle présente une pointe effilée qui est susceptible d'agresser les tissus en cas d'une éventuelle traversée de l'os.

Pour pallier au premier inconvénient il a été proposé dans le document FR-A-2 721 819, un dispositif de cheville ou vis de blocage présentant une tête fraiseuse permettant qu'elle soit enfouie dans la plaque d'osthéosynthèse ou dans la corticale, et une extrémité autoforeuse consistant en un plat positionné transversalement, découpé en points, dont les deux pans, qui forment un angle obtus, sont biseautés de façon symétrique.

Si cette vis constitue un progrès par rapport à celle du document FR-A-2 625 430, elle n'en présente pas moins également des inconvénients.

En effet, la pointe de cette vis ne permet pas, en fonction de la qualité de la matière osseuse, un forage performant, de sorte que l'on observe fréquemment une rupture prématurée de la partie sécable lors de l'opération en augmentant la résistance à la rupture, mais dans ce cas, il n'y a plus rupture automatique en fin de vissage.

D'autre part, la matière osseuse est difficilement mesurable en préopératoire, elle ne peut être qu'estimée, et comme il est nécessaire d'utiliser une vis qui soit la plus longue possible, il est fréquent que la pointe de cette dernière fasse saillie de l'autre côté, or cette pointe est très effilée et elle agresse les tissus environnants lors des mouvements, le patient ressent sa présence et en souffre.

D'autre part encore, le filetage de cette vis est d'un pas très court, de l'ordre de 0,8 mm, et il est à filet triangulaire asymétrique avec un flanc proximal radial à l'axe de la vis et un flanc distal faisant un angle de 60 degrés, de sorte que cette vis, destinée initialement à la stomatologie pour des os très durs, a tendance à mal fonctionner dans l'os cortico-spongieux.

On a décrit dans le document EP-A-0 699 420, une cheville autoforeuse et autotaraudeuse à embout de maintien sécable, comportant une extrémité autoforeuse d'une tige filetée qui est un plat positionné transversalement et découpé en une pointe dont les deux pans sont biseautés de façon symétrique pour former un angle obtus. Ce document est la base du préambule de la revendication 1.

Un tel dispositif ne permet pas un forage et un taraudage corrects lors de la mise en place de la cheville, et est susceptible d'agresser les tissus en cas d'une éventuelle traversée de l'os.

La présente invention a pour but de proposer un dispositif de vis sécable permettant de remédier à ces divers inconvénients.

Le dispositif de vis sécable, objet de la présente invention, est conforme au libellé de la revendication 1.

Le dispositif de vis sécable, objet d'une réalisation possible de la présente invention, est du type comportant une tige filetée prolongée d'une tête fraisée solidarisée, par l'intermédiaire d'une zone étranglée de moindre résistance à un couple donné, à un embout serrable dans le mandrin d'un outil de vissage, ladite tige filetée comportant une extrémité distale caractérisé en ce qu'il comporte une pointe, ayant la forme d'un cône d'un angle très ouvert, par exemple égal à environ 60 degrés par rapport à l'axe longitudinal XX de la tige filetée, l'extrémité distale de ladite tige filetée comportant trois entailles longitudinales de taraudage pratiquées à la fois dans le filetage et dans la racine de la tige filetée, comprenant chacune deux pans faisant entre eux un angle droit, dont l'un, constituant le bord d'attaque de la matière osseuse, est dans le plan radial à l'axe de ladite tige filetée, et en ce que ladite pointe de l'extrémité distale est coupante, de façon à présenter des qualités de pénétration tout en n'agressant pas les tissus environnants en cas d'une éventuelle traversée de l'os.

Selon un autre caractère avantageux du dispositif selon l'invention, ladite pointe coupante de l'extrémité distale comporte trois entailles de forage comportant chacune deux pans.

Selon un autre caractère avantageux du dispositif selon l'invention, les trois entailles de forage pratiquées dans la pointe sont situées chacune dans le prolongement de l'une desdites entailles de taraudage.

Selon un autre caractère avantageux du dispositif selon l'invention, l'un des pans de chaque entaille de forage pratiquée dans la pointe est dans le prolongement du pan constituant le bord d'attaque de l'entaille de taraudage, tandis que l'autre pan fait un angle ouvert avec l'autre pan de l'entaille de taraudage, de manière que l'arête constituant le fond de l'entaille de forage débouche à proximité de l'extrémité de ladite pointe.

Selon une autre caractéristique avantageuse du dispositif selon l'invention, la tête présente une partie fraisée bordée supérieurement et périphériquement d'une collerette de faible épaisseur, dont la face inférieure est perpendiculaire à l'axe de la vis.

Selon une autre caractéristique avantageuse de l'invention, la tête comporte trois encoches, destinées au maniement de la vis, au moyen d'un outil adapté, après rupture de la zone de moindre résistance.

Selon un mode de réalisation de l'invention, le dispositif est tel que la pointe de l'extrémité distale comporte un prolongement en tronc de cône d'angle égal à environ 60° par rapport à l'axe longitudinal XX de la tige filetée et une partie d'extrémité en pointe d'autocentrage.

La zone étranglée comporte une jonction avec la tête fraisée formant un angle vif permettant une cassure nette entre la zone étranglée et la tête fraisée.

Le diamètre externe de la tige filetée est d'environ 2mm.

Les avantages et les caractéristiques de la présente invention ressortiront plus clairement de la description qui suit et se rapporte au dessin annexé, qui représente un mode de réalisation non limitatif.

La Figure 1 représente une vue en plan d'un dispositif de vis sécable selon la présente invention.

La Figure 2 représente une vue de face de l'extrémité distale du dispositif de la Figure 1.

La Figure 3 représente une vue partielle en perspective du dispositif de la Figure 1.

La Figure 4 représente une vue en plan d'un autre mode de réalisation du dispositif de vis sécable selon la présente invention.

La Figure 5 une vue en plan partielle de la zone étranglée du dispositif de vis sécable selon la présente invention.

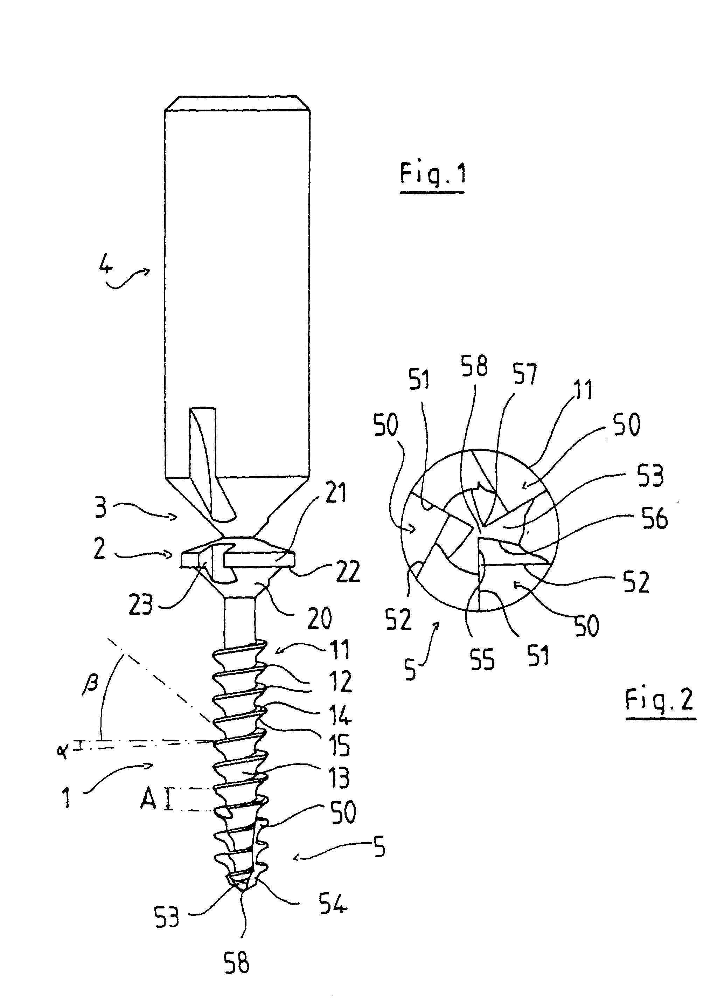

Si on se réfère à la Figure 1, on peut voir qu'un dispositif de vis sécable selon la présente invention comporte une tige filetée 1, surmontée d'une tête fraisée 2 solidaire, par l'intermédiaire d'une zone 3 de moindre résistance à un couple donné, d'un embout 4 d'adaptation dans le mandrin d'un outil de vissage électrique ou pneumatique.

La tige filetée 1 présente à son extrémité proximale une partie 10 non filetée autorisant un coulissement, lors de la fixation d'une plaque d'ostéosynthèse, de manière à permettre une compression de cette dernière contre la corticale.

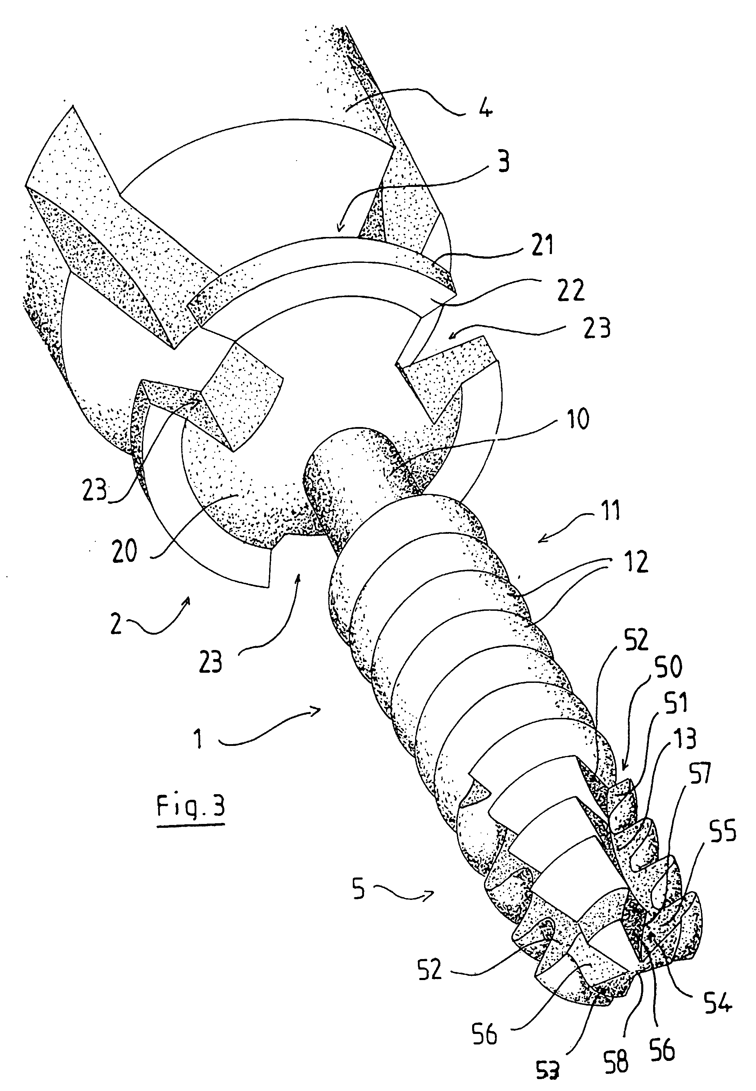

La tige filetée 1 comporte un filetage 11 et une extrémité 5 autoforeuse et autotaraudeuse qui va être décrite maintenant à l'aide des Figures 2 et 3.

Le taraudage est réalisé au moyen de trois entailles 50 pratiquées longitudinalement dans les filets 12 et dans la racine 13 de la tige filetée, et comportant chacune deux pans 51 et 52 faisant entre eux un angle droit, le pan 51, qui constitue le bord d'attaque, étant dans un plan radial à l'axe de la tige filetée 1.

Le forage est réalisé au moyen d'une pointe 53 en forme d'un cône d'un angle de 120 degrés dans laquelle sont pratiquées trois entailles 54, chacune dans le prolongement d'une entaille 5, et comportant chacune deux pans 55 et 56, le pan 55 étant dans le même plan que le pan 51, tandis que le pan 56 est oblique, à savoir qu'il fait un angle ouvert avec le pan 52, l'arête 57 constituant le fond de l'entaille 54 débouchant à proximité de l'extrémité 58 de la pointe 53.

Les entailles 50 sont très longues, elles sont pratiquées dans plusieurs filets 12, au moins trois, de manière à permettre l'évacuation des copeaux d'os, notamment lors du perçage et du taraudage de la corticale.

On notera que les extrémités proximales des pans 52 et 56 sont légèrement courbes alors que les pans 51 et 55 sont plans, ceci en raison de la réalisation des entailles 50 et 54 par meulage.

La pointe 53, qui peut être assimilée à la pointe d'un foret, est très coupante, de sorte que dans le cas d'une éventuelle traversée de l'os, elle n'agresse pas les tissus environnants.

Les qualités de pénétration de la pointe 53 sont telles qu'il est possible de diminuer la résistance de la zone de rupture 3 par rapport à celle des dispositifs de vis existants, ce qui permet d'éviter une rupture prématurée.

Sur les Figures 1 et 3, on peut voir que la tête 2 présente une partie fraisée 20 bordée supérieurement et périphériquement d'une collerette 21 de faible épaisseur, dont la face inférieure 22 est perpendiculaire à l'axe de la vis.

La collerette 21 offre une surface d'appui importante, ce qui permet d'arrêter l'enfouissement de la tête dans la corticale, et d'assurer la rupture de la zone 3 en fin de vissage.

D'autre part, la tête 2 comporte trois encoches 23 permettant de manoeuvrer la vis, au moyen d'un outil adapté, après rupture de la zone 3, soit pour finir le vissage, soit pour dévisser la vis.

Le filetage 11 est adapté à l'os cortico-spongieux, c'est-à-dire que, comme on peut le voir sur la Figure 1, le pas A est relativement important, tandis que les flancs proximaux 14 et distaux 15 des filets 12 font entre eux un angle faible.

A titre d'exemple, pour une vis, hors embout 4, de 11 mm, le pas A est de 1,8 mm, et les flancs proximaux 14 font un angle α de 3 degrés par rapport à un axe radial, tandis que les flancs distaux 15 font un angle β de 35 degrés par rapport à ce même axe.

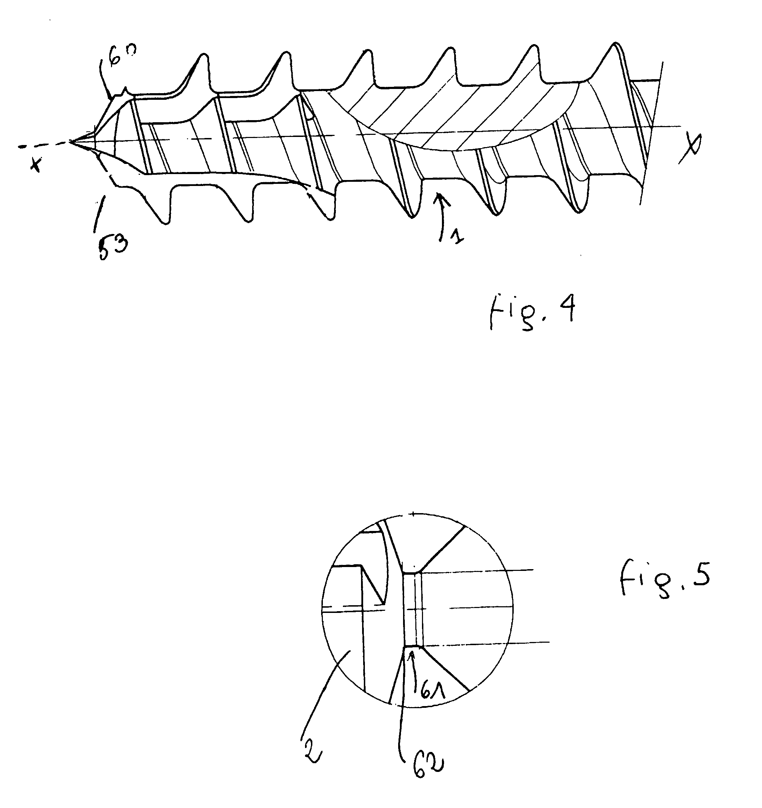

Sur la Figure 4, on voit que la pointe 53 de l'extrémité distale 5 comporte une partie intermédiaire 60 en tronc de cône d'angle égal à environ 60 ° par rapport à l'axe longitudinal XX de la tige filetée 1 et une partie d'extrémité en pointe d'autocentrage. Cette pointe d'autocentrage permet de garder l'orientation de la vis lorsqu'elle est en contact avec des fragments osseux et que l'on commence la rotation du dispositif.

Sur la Figure 5, on a représenté une vue agrandie de la zone étranglée 3 qui comporte une jonction 61 avec la tête fraisée 2. Cette jonction 61 forme un angle vif en 62 permettant une cassure nette entre la zone étranglée 3 et la tête fraisée 2. De ce fait, la zone de rupture est située à la tête, ce qui permet d'éviter les débris de matière du dispositif qui viendrait en conflit avec les tissus mous.

De préférence, le diamètre externe de la tige filetée 1 est d'environ 2mm.

## Revendications

1. Dispositif de vis sécable pour plaque d'ostéosynthèse ou pour la coaptation de deux fragments d'os, comportant une tige filetée (1) prolongée d'une tête fraisée (2) solidarisée, par l'intermédiaire d'une zone étranglée (3) de moindre résistance à un couple donné, à un embout (4) serrable dans le mandrin d'un outil de vissage, ladite tige filetée (1) comportant une extrémité distale (5) **caractérisé en ce qu'**il comporte une pointe (53) ayant la forme d'un cône, l'extrémité distale (5) de ladite tige filetée (1) comportant trois entailles longitudinales (50) de taraudage pratiquées à la fois dans le filetage (11) et dans la racine (13) de la tige filetée (1) et comprenant chacune deux pans (51, 52) faisant entre eux un angle droit, dont l'un (51), constituant le bord d'attaque de la matière osseuse, est dans un plan radial à l'axe de ladite tige filetée, trois entailles de forage (54) étant pratiquées dans ladite pointe (53) de l'extrémité distale, lesdites trois entailles de forage (54) étant situées chacune dans le prolongement de l'une desdites entailles de taraudage (50) et comportant chacune deux pans (55, 56) dont l'un (55) est dans le prolongement du pan (51) constituant le bord d'attaque de l'entaille de taraudage (50), tandis que l'autre pan (56) fait un angle ouvert avec l'autre pan (52) de l'entaille de taraudage (50), de manière à ce que l'arête (57) constituant le fond de l'entaille de forage (54) débouche à proximité de l'extrémité (58) de ladite pointe (53).

2. Dispositif selon la revendication 1 **caractérisé en ce que** la tête (2) présente une partie fraisée (20) bordée supérieurement et périphériquement d'une collerette (21) de faible épaisseur, dont la face inférieure (22) est perpendiculaire à l'axe de la vis.

3. Dispositif selon l'une des revendications précédentes **caractérisé en ce que** la tête (2) comporte trois encoches (23) de manipulation de la vis, au moyen d'un outil adapté, après rupture de la zone de moindre résistance (3).

4. Dispositif selon l'une quelconque des revendications précédentes **caractérisé en ce que** chacune des entailles de taraudage (50) est pratiquée dans au moins trois filets (12).

5. Dispositif selon l'une quelconque des revendications précédentes **caractérisé en ce que** les flanc proximaux (14) des filets (12) du filetage (11) font un angle (α) de 3 degrés par rapport à un axe radial, tandis que les flancs distaux (15) font un angle (β) de 35 degrés par rapport audit axe.

6. Dispositif selon l'une quelconque des revendications précédentes **caractérisé en ce que** la pointe (53) de l'extrémité distale (5) comporte une partie intermédiaire (60) en tronc de cône et une partie d'extrémité en pointe d'auto centrage.

7. Dispositif selon l'une quelconque des revendications précédentes **caractérisé en ce que** la zone étranglée (3) comporte une jonction avec la tête fraisée (2) formant un angle vif permettant une cassure nette entre la zone étranglée (3) et la tête fraisée (2).

8. Dispositif selon l'une quelconque des revendications précédentes **caractérisé en ce que** le diamètre externe de la tige filetée (1) est de 2 mm.

## Claims

1. Divisible screw device for osteosynthesis plate or for co-aptation of two fragments of bone, comprising a threaded rod (1) extended by a milled head (2) which by way of a constricted zone of least resistance (3) at a given torque is attached to an end piece (4) which can be clamped in the mandrel of a screwing tool, said threaded rod (1) comprising a distal end (5), **characterised in that** it has a tip (53) in the form of a cone, where the distal end (5) of said threaded rod (1) has three longitudinal tapping grooves (50) produced both in the thread (11) and in the root (13) of the threaded rod (1) and each comprising two flat sides (51, 52) forming between them a right angle, the one (51) of which constituting the leading edge attacking the bone material lies in a plane radial to the axis of the said threaded rod, where three drilling grooves (54) are produced in the said tip (53) of the distal end, said three drilling grooves (54) each being located in the extension of one of the said tapping grooves (50) and each comprising two flat sides (55, 56), one (55) of which lies in the extension of the flat side (51) constituting the leading edge of the tapping groove (50) whereas the other flat side (56) forms an open angle with the other flat side (52) of the tapping groove (50) such that the edge (53) forming the base of the drilling groove (54) terminates close to the end (58) of said tip (53).

2. Device according to claim 1, **characterised in that** the head (2) has a milled part (20) edged at the top and peripherally with a collar (21) of low thickness, the lower face (22) of which is perpendicular to the axis of the screw.

3. Device according to any of the previous claims, **characterised in that** the head (2) has three notches (23) for handling the screw by means of a suitable tool after breakage of the zone of least resistance (3).

4. Device according to any of the previous claims, **characterised in that** each of the tapping grooves (50) is produced over at least three threads (12).

5. Device according to any of the previous claims, **characterised in that** the proximal flanks (14) of the threads (12) of the thread (11) form an angle (α) of 3 degrees in relation to the radial axis, whereas the distal flanks (15) form an angle (β) of 35 degrees in relation to the said axis.

6. Device according to any of the previous claims, **characterised in that** the tip (53) of the distal end (5) has a frustoconical intermediate part (60) and a self-centring pointed end part.

7. Device according to any of the previous claims, **characterised in that** the constricted zone (3) comprises a junction with the milled head (2) forming an acute angle allowing a clean break between the constricted zone (3) and the milled head (2).

8. Device according to any of the previous claims, **characterised in that** the external diameter of the threaded rod (1) is 2 mm.

## Patentansprüche

1. Trennbare Vorrichtung für eine Knochenplatte oder zur Fixierung zweier Knochenfragmente mit einem Gewindeschaft (1), der verlängert ist mit einem gefrästen Kopf (2), der über einen verjüngten Bereich (3) mit geringerem Widerstand gegen eine bestimmte Belastung verbunden ist mit einem Aufsatz, der in das Spannfutter eines Schraubwerkzeugs gezwängt werden kann, wobei der Gewindeschaft (1) ein äußeres Ende (5) aufweist, **dadurch gekennzeichnet, daß** sie eine Spitze (53) aufweist mit der Form eines Konus, wobei das äußere Ende (5) des Gewindeschafts (1) drei longitudinale Einschnitte (50) zum Schneiden aufweist, die zusammen in das Gewinde (11) und in den Kern (13 des Gewindeschafts (1) gefertigt sind und jeweils zwei Schenkel (51, 52) aufweisen, die zwischen sich einen rechten Winkel bilden und von denen einer (51), der die Schneidkante für die Knochenmaterie bildet, in einer radialen Ebene zur Achse des Gewindeschafts (1) ist, wobei drei Einschnitte zum Bohren (54) in die Spitze (53) des äußeren Endes gefertigt sind und die drei Einschnitte zum Bohren (54) jeweils in der Verlängerung einer der Einschnitte (50) zum Schneiden angeordnet sind und jeder zwei Schenkel (55, 56) aufweist, von denen einer (55) in der Verlängerung des Schenkels (51) ist, der die Schneidkante des Einschnitts (50) zum Schneiden bildet, wohingegen der andere Schenkel (56) einen offenen Winkel bildet mit dem anderen Schenkel (52) des Einschnitts (50) zum Schneiden, so daß die Kante (57), die den Boden für den Einschnitt zum Bohren (54) bildet, nahe dem Ende (58) der Spitze (53) austritt.

2. Vorrichtung gemäß Anspruch 1, **dadurch gekennzeichnet, daß** der Kopf (2) einen gefrästen Abschnitt (20) aufweist, der oberhalb und am Umfang mit einem Rand (21) geringer Dicke umgeben ist, dessen untere Seite senkrecht ist zur Achse der Schraube.

3. Vorrichtung gemäß einem der vorhergehenden Ansprüche, **dadurch gekennzeichnet, daß** der Kopf (2) drei Einkerbungen (23) zur Handhabung der Schraube mit einem passenden Werkzeug aufweist nach dem Bruch des Bereichs (3) mit geringerem Widerstand.

4. Vorrichtung gemäß einem der vorhergehenden Ansprüche, **dadurch gekennzeichnet, daß** jede der drei Einschnitte zum Schneiden (50) in mindestens drei Gewindegänge (12) gefertigt ist.

5. Vorrichtung gemäß einem der vorhergehenden Ansprüche, **dadurch gekennzeichnet, daß** die anliegenden Seiten (14) der Gewindegänge (12) des Gewindes 11 einen Winkel (α) von 3 Grad relativ zu einer radialen Achse bilden, wohingegen die abgewandten Seiten (15) einen Winkel (β) von 35 Grad relativ zu dieser Achse bilden.

6. Vorrichtung gemäß einem der vorhergehenden Ansprüche, **dadurch gekennzeichnet, daß** die Spitze (53) des äußeren Endes (5) einen Zwischenabschnitt (60) mit einem konischen Stumpf und einen äußeren Abschnitt mit selbstzentrierender Spitze aufweist.

7. Vorrichtung gemäß einem der vorhergehenden Ansprüche, **dadurch gekennzeichnet, daß** der verjüngte Bereich (3) eine Verbindung mit dem gefrästen Kopf (2) aufweist, die einen spitzen Winkel bildet, der einen glatten Bruch zwischen dem verjüngten Bereich (3) und dem gefrästen Kopf (2) ermöglicht.

8. Vorrichtung gemäß einem der vorhergehenden Ansprüche, **dadurch gekennzeichnet, daß** der äußere Durchmesser des Gewindeschafts (1) 2 mm beträgt.
